# EUROPÄISCHE PATENTSCHRIFT

(11) **EP 2 004 123 B1**
(45) Veröffentlichungstag und Bekanntmachung des Hinweises auf die Patenterteilung: **13.10.2010**
(21) Anmeldenummer: 07785697.9
(22) Anmeldetag: 07.08.2007
(51) Int. Cl.: A61G 5/10

(54) **ROLLSTUHL, KRANKENSTUHL ODER DGL.**
WHEELCHAIR, INVALID CHAIR OR THE LIKE
FAUTEUIL ROULANT, CHAISE POUR INVALIDE ET D'AUTRES

(30) Priorität: 17.08.2006 DE 102006038630
(43) Veröffentlichungstag der Anmeldung: 24.12.2008
(73) Patentinhaber: Soehnle Professional GmbH & Co. KG, 71522 Backnang (DE)
(72) Erfinder: GERSTER, Stephan, 53323 Wachtberg-Pech (DE)
(86) Internationale Anmeldenummer: PCT/DE2007/001396
(87) Internationale Veröffentlichungsnummer: WO 2008/019655

(56) Entgegenhaltungen:
- JP-A- 2000 225 101
- US-A1- 2005 165 325
- US-A1- 2005 178 590

## Beschreibung

Die vorliegende Erfindung betrifft einen Rollstuhl, Krankenstuhl oder dgl., mit einem Rahmen, ggf. mit dem Rahmen verbundenen Armlehnen, einem vom Rahmen getragenen Sitz und Rädern, wobei zwischen dem Rahmen und/oder dem Sitz und den Rädern, vorzugsweise in oder an den Radachsen, Wägezellen zur Gewichtsermittlung vorgesehen sind und wobei die Messsignale in einer eine Stromversorgung, einen Prozessor und ggf. einen Speicher umfassenden Auswerteeinheit ausgewertet und die aufbereiteten Daten, insbesondere das Gewicht der im Rollstuhl sitzenden Person, auf einem Display angezeigt werden.

Rollstühle, Krankenstühle etc. mit integrierter Waage, d.h. mit integrierter Gewichtsermittlung der darin sitzenden Person, sind sei geraumer Zeit aus der Praxis bekannt. Die zum Wiegen erforderlichen Wägezellen wirken dort üblicherweise zwischen dem Rahmen bzw. dem Sitz und den Rädern. Entsprechend ausgestattete Krankenstühle werden meist zur Gewichtskontrolle von Personen eingesetzt, denen das Stehen schwer fällt oder für bettlägrige Personen. Die Handhabung solcher Krankenstühle ist unkompliziert, zumal es lediglich erforderlich ist, dass sich die Person auf den Stuhl setzt. Ähnlich wie bei modernen Waagen, ist in einem Gehäuse ein Prozessor nebst Speicher vorgesehen, wobei die ausgewählten und gegebenenfalls aufbereiteten Daten über ein LCD-Display angezeigt werden. Die Bedienung entspricht der einer handelsüblichen Waage mit entsprechender Ausstattung.

Neben dem Gewicht besteht meist Bedarf nach der Ermittlung weiterer körperspezifischer Daten, wie dies bei Waagen mit integrierter Fettmessfunktion der Fall üblich ist. Solche Waagen mit integrierter Fettmessfunktion arbeiten regelmäßig nach der sog. Bioimpedanzmethode. Sie sind auf der Wiegeplatte bzw. auf der Standfläche für die zu wiegende Person mit elektrisch leitenden Elektroden ausgestattet, die wiederum mit einem Prüfstrom gespeist werden. Es wird der Prüfstrom durch den Körper des Benutzers geleitet, woraus sich der körperspezifische elektrische Widerstand bestimmen lässt. Aus diesem Körperwiderstand und dem über die Wägezelle ermittelten Gewicht sowie ggf. aus weiteren körperspezifischen Daten - ggf. Geschlecht, Alter, etc. - lässt sich der Fettgehalt ermitteln.

Die aus der Praxis bekannten Waagen mit integrierter Fettmessfunktion sind jedoch für Rollstuhlfahrer und bettlägrige Personen problematisch, da eine Positionierung dieser Personen auf entsprechenden Waagen nahezu unmöglich ist.

Der vorliegenden Erfindung liegt daher die Aufgabe zugrunde, eine gattungsbildenden Rollstuhl, Krankenstuhl oder dgl. derart auszugestalten und weiterzubilden, dass neben dem Gewicht die Ermittlung weiterer körperspezifischer Informationen möglich ist.

Der erfindungsgemäße Rollstuhl, Krankenstuhl oder dgl. löst die voranstehende Aufgabe durch die Merkmale des Patentanspruchs 1. Danach ist der gattungsbildende Rollstuhl, Krankenstuhl oder dgl. **dadurch gekennzeichnet, dass** zur Ermittlung weiterer körperspezifischer Informationen, insbesondere zur Ermittlung des Körperfetts (des Fett-, Wasser- und Muskelanteils), eine zusätzliche Messeinrichtung mit Elektroden vorgesehen ist, wobei die Elektroden dem Rahmen oder den Armlehnen oder zusätzlichen Griffen zugeordneten sind und wobei die diesbezüglichen Messwerte der Auswerteeinheit zugeführt werden.

Erfindungsgemäß ist erkannt worden, dass es möglich ist, in den Rollstuhl die Ermittlung weiterer körperspezifischer Informationen zu integrieren, und zwar zusätzlich zu der Ermittlung des Körpergewichts. So ist es denkbar, an der im Rollstuhl bzw. im Krankenstuhl sitzenden Person die Ermittlung des Körperfetts, d.h. des Fett-, Wasser- und Muskelanteils im Körper, vorzunehmen. Dazu ist eine zusätzliche Messeinrichtung mit Elektroden vorgesehen, wobei die Elektroden dem Rahmen oder den Armlehnen oder zusätzlichen Griffen zugeordnet sind und wobei die diesbezüglichen Messwerte einer Auswerteeinheit zugeführt werden, die auch zur Gewichtsermittlung dient.

In erfindungsgemäßer Weise ist erkannt worden, dass die Ermittlung weiterer körperspezifischer Informationen am bzw. im Rollstuhl möglich ist, nämlich dann, wenn eine zusätzliche Messeinrichtung mit Elektroden vorgesehen ist, die sich beispielsweise zur Anwendung der Bioimpedanzmethode eignet. Für diese Methode lassen sich grundsätzlich alle Faktoren ermitteln, die eine Korrelation mit dem ermittelbaren Körperwiderstand und dem ermittelten Gewicht haben. So ist es grundsätzlich denkbar, dass aus den Messwerten zahlreiche Informationen in Bezug auf den gesundheitlichen Zustand der jeweiligen Personen abgeleitet werden.

Die Elektroden lassen sich grundsätzlich dem meist aus Metall bestehenden Rahmen des Rollstuhls zuordnen. Insbesondere ist es denkbar, dass die Elektroden den Armlehnen zugeordnet werden, so dass beim Kontakt der Hand oder des Arms mit den Armlehnen eine Messung nach der Bioimpedanzmethode erfolgen kann.

Ebenso ist es dankbar, dass die Elektroden zusätzlichen Griffen zugeordnet sind, die sich an den Rahmen des Rollstuhl anstecken lassen oder die separat im Sinne von leichten Hanteln oder dgl. handhabbar sind.

Auch ist es denkbar, dass die Elektroden einer zusätzlichen Platte - ähnlich einer Schreibunterlage - zugeordnet sind, wobei die Platte mit dem Material der Elektroden in zwei voneinander getrennten Zonen versehen ist. Die Messung lässt sich vornehmen, indem die zu vermessende Person die Hände oder die Unterarme auf die voneinander getrennten Zonen legt und somit einen elektrischen Kontakt zum Körper herstellt.

In besonders vorteilhafter Weise sind die Elektroden durch Oberflächtenbeschichtung der die Elektroden tragenden Teile erzeugt, insbesondere durch Bedampfen mittels Metall oder einer sonstigen elektrisch leitenden Schicht.

Die Elektroden bzw. die die Elektroden tragenden Teile können kontaktbehaftet oder kontaktlos mit der Auswerteeinheit verbunden sein. Im Falle einer kontaktlosen Ausgestaltung ist die Vorkehrung eines Sender-/Empfängersystems erforderlich.

Des Weiteren ist es von Vorteil, wenn die Auswerteeinheit und ggf. die die Elektroden tragenden Teile vom Rollstuhl abnehmbar sind. Somit lässt sich die Messeinrichtung universell an unterschiedlichen Rollstühlen zum Einsatz bringen.

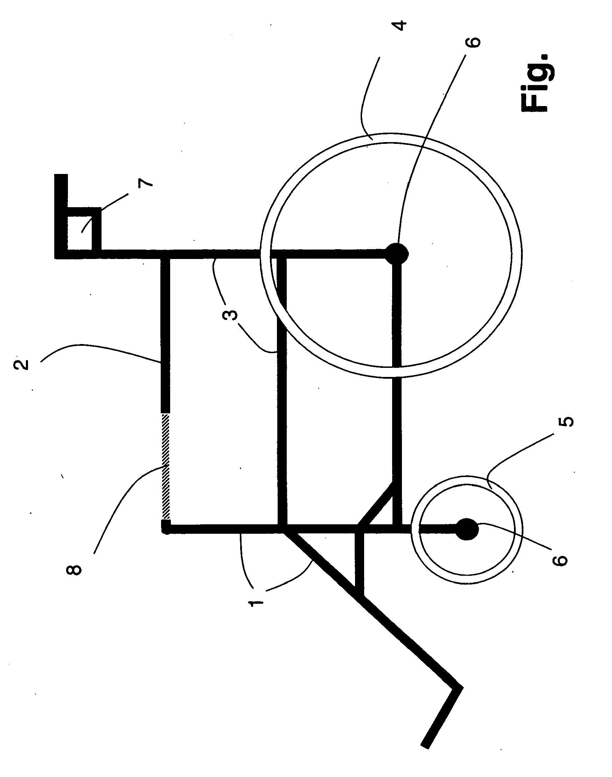

Es gibt nun verschiedene Möglichkeiten, die Lehre der vorliegenden Erfindung in vorteilhafter Weise auszugestalten und weiterzubilden. Dazu ist einerseits auf die nachgeordneten Ansprüche, andererseits auf die nachfolgende Erläuterung eines bevorzugten Ausführungsbeispiels der Erfindung anhand der Zeichnung zu verweisen. In Verbindung mit der Erläuterung des bevorzugten Ausführungsbeispiels der Erfindung anhand der Zeichnung werden auch im Allgemeinen bevorzugte Ausgestaltungen und Weiterbildungen der Lehre erläutert. In der Zeichnung zeigt
- die einzige Figur: in einer schematischen Ansicht ein Ausführungsbeispiel eines erfindungsgemäßen Rollstuhls mit integrierter Waage und Körperfettmesseinrichtung.

Die einzige Figur zeigt in schematischer Ansicht einen Rollstuhl, der im Wesentlichen einen Rahmen 1, mit dem Rahmen 1 verbundene Armlehnen 2, ein vom Rahmen 1 getragenen Sitz 3 und zwei große Räder 4 hinten sowie zwei kleine Räder 5 vorne aufweist, wobei die kleinen Räder 5 als Lenkräder dienen.

In der Figur nicht erkennbare Wägezellen dienen zur Gewichtsermittlung und sind den Radachsen zugeordnet.

Des Weiteren ist - bei dem hier gewählten Ausführungsbeispiel eine Auswerteeinheit 7 dem Rückenteil des Sitzes 3 zugeordnet, die einen Prozessor und einen Speicher nebst Stromversorgung umfasst und zur Auswertung bzw. Aufbereitung der Messsignale dient. Des Weiteren umfasst die Auswerteeinheit 7 ein Display zur Anzeige der Messergebnisse.

In erfindungsgemäßer Weise ist zur Ermittlung weiterer körperspezifischer Informationen, insbesondere zur Ermittlung des Körperfetts, d.h. des Fett-, Wasser- und Muskelanteils des Körpers, eine zusätzliche Messeinrichtung vorgesehen. Diese Messeinrichtung umfasst Elektroden 8, die bei dem hier gewählten Ausführungsbeispiel in die Armlehnen 2 integriert sind. Ebenso können zusätzliche Griffe, eine Platte oder sonstige Träger für die Elektroden vorgesehen sein, wobei zwei separate Elektroden erforderlich sind, die zur Kontaktierung der Hände oder Unterarme der jeweiligen Person dienen. Ebenso wäre es denkbar, Elektroden in Form von Manschetten vorzusehen, die man um die Handgelenke oder um die Fußgelenke legen bzw. dort entsprechend befestigen kann. Eine Anbringung mittels Gummiband oder Klettverschluss ist denkbar.

Schließlich sei angemerkt, dass das voranstehend erörterte Ausführungsbeispiel lediglich der beispielhaften Erörterung der beanspruchten Lehre dient, diese jedoch nicht auf das Ausführungsbeispiel einschränken.

## Patentansprüche

1. Rollstuhl, Krankenstuhl oder dgl., mit einem Rahmen (1), ggf. mit dem Rahmen (1) verbundenen Armlehnen (2), einem vom Rahmen (1) getragenen Sitz (3) und Rädern (4, 5), wobei zwischen dem Rahmen (1) und/oder dem Sitz (3) und den Rädern (4, 5), vorzugsweise in oder an den Radachsen (6), Wägezellen zur Gewichtsermittlung vorgesehen sind und wobei die Messsignale in einer eine Stromversorgung, einen Prozessor und ggf. einen Speicher umfassenden Auswerteeinheit (7) ausgewertet und die aufbereiteten Daten, insbesondere das Gewicht der im Rollstuhl sitzenden Person, auf einem Display angezeigt werden,
**dadurch gekennzeichnet, dass** zur Ermittlung weiterer körperspezifischer Informationen, insbesondere zur Ermittlung des Körperfetts, des Fett-, Wasser- und Muskelanteils, eine zusätzliche Messeinrichtung mit Elektroden (8) vorgesehen ist, wobei die Elektroden (8) dem Rahmen (1) oder den Armlehnen (2) oder zusätzlichen Griffen zugeordneten sind und wobei die diesbezüglichen Messwerte der Auswerteeinheit (7) zugeführt werden.

2. Rollstuhl nach Anspruch 1, **dadurch gekennzeichnet, dass** die Elektroden (8) dem Rahmen (1) zugeordnet sind.

3. Rollstuhl nach Anspruch 1, **dadurch gekennzeichnet, dass** die Elektroden (8) den Armlehnen (2) zugeordnet sind.

4. Rollstuhl nach Anspruch 1, **dadurch gekennzeichnet, dass** die Elektroden (8) zusätzlichen Griffen zugeordneten sind.

5. Rollstuhl nach Anspruch 1, **dadurch gekennzeichnet, dass** die Elektroden (8) einer zusätzlichen Platte zugeordnet sind.

6. Rollstuhl nach Anspruch 5, **dadurch gekennzeichnet, dass** die Platte mit dem Material der Elektroden (8) in zwei voneinander getrennten Zonen versehen ist.

7. Rollstuhl nach einem der Ansprüche 1 bis 6, **dadurch gekennzeichnet, dass** die Elektroden (8) durch Oberflächenbeschichtung der jeweiligen Teile, insbesondere durch Bedampfen, erzeugt sind.

8. Rollstuhl nach einem der Ansprüche 1 bis 7, **dadurch gekennzeichnet, dass** die Elektroden (8) kontaktbehaftet oder kontaktlos mit der Auswerteeinheit (7) verbunden sind.

9. Rollstuhl nach einem der Ansprüche 1 bis 8, **dadurch gekennzeichnet, dass** die Auswerteeinheit (7) und ggf. die die Elektroden (8) tragenden Teile vom Rollstuhl abnehmbar ist/sind.

## Claims

1. Wheelchair, invalid chair or the like, with a frame (1), possibly with armrests (2) connected to the frame (1), a seat (3) supported by the frame (1) and wheels (4, 5), in which weighing cells for determining weight are provided between the frame (1) and/or the seat (3) and the wheels (4, 5), preferably in or on the wheel axles (6), and in which the measurement signals are evaluated in an evaluation unit (7) comprising a power supply, a processor and possibly a storage medium and the processed data, more particularly the weight of the person sitting in the wheelchair, is shown on a display,
**characterized in that** provision is made for an additional measuring apparatus with electrodes (8) for determining further body-specific information, more particularly for determining the body fat, the fat, water and muscle components, in which the electrodes (8) are assigned to the frame (1) or the armrests (2) or additional handles and in which the measured values relating thereto are supplied to the evaluation unit (7).

2. Wheelchair according to Claim 1, **characterized in that** the electrodes (3) are assigned to the frame (1).

3. Wheelchair according to Claim 1, **characterized in that** the electrodes (8) are assigned to the armrests (2).

4. Wheelchair according to Claim 1, **characterized in that** the electrodes (8) are assigned to additional handles.

5. Wheelchair according to Claim 1, **characterized in that** the electrodes (8) are assigned to an additional plate.

6. Wheelchair according to Claim 5, **characterized in that** the plate with the material of the electrodes (8) is provided in two mutually separate zones.

7. Wheelchair according to one of Claims 1 to 6, **characterized in that** the electrodes (8) are produced by surface coating of the respective parts, in particular by evaporation.

8. Wheelchair according to one of Claims 1 to 7, **characterized in that** the electrodes (8) are connected to the evaluation unit (7) with or without contact.

9. Wheelchair according to one of Claims 1 to 8, **characterized in that** the evaluation unit (7) and, where applicable, the parts supporting the electrodes (8) can be removed from the wheelchair.

## Revendications

1. Fauteuil roulant, chaise de malade ou similaire, comportant un châssis (1), éventuellement des accoudoirs (2) reliés au châssis (1), un siège (3) supporté par le châssis (1) et des roues (4, 5), des cellules de pesage permettant de déterminer le poids étant prévues entre le châssis (1) et/ou le siège (3) et les roues (4, 5), de préférence dans ou sur les axes des roues (6) et les signaux de mesure étant exploités dans une unité d'exploitation (7) comprenant une alimentation en courant, un processeur et éventuellement une mémoire, et les données élaborées, notamment le poids de la personne assise dans le fauteuil roulant, étant affichées sur un afficheur,
**caractérisé en ce que**, pour déterminer d'autres informations spécifiques au corps, notamment pour déterminer l'adiposité corporelle, la proportion de graisse, d'eau et de muscles, il est prévu un dispositif de mesure supplémentaire à électrodes (8), les électrodes (8) étant associées au châssis (1) ou aux accoudoirs (2) ou à des poignées supplémentaires et les valeurs de mesure corrélatives étant acheminées vers l'unité d'exploitation (7).

2. Fauteuil roulant selon la revendication 1, **caractérisé en ce que** les électrodes (8) sont associées au châssis (1).

3. Fauteuil roulant selon la revendication 1, **caractérisé en ce que** les électrodes (8) sont associées aux accoudoirs (2).

4. Fauteuil roulant selon la revendication 1, **caractérisé en ce que** les électrodes (8) sont associées à des poignées supplémentaires.

5. Fauteuil roulant selon la revendication 1, **caractérisé en ce que** les électrodes (8) sont associées à une plaque supplémentaire.

6. Fauteuil roulant selon la revendication 5, **caractérisé en ce que** la plaque est pourvue du matériau des électrodes (8) dans deux zones séparées l'une de l'autre.

7. Fauteuil roulant selon une des revendications 1 à 6, **caractérisé en ce que** les électrodes (8) sont créées par revêtement de la surface des pièces respectives, notamment par vaporisation.

8. Fauteuil roulant selon une des revendications 1 à 7, **caractérisé en ce que** les électrodes (8) sont collées par colle contact ou reliées sans contact à l'unité d'exploitation (7).

9. Fauteuil roulant selon une des revendications 1 à 8, **caractérisé en ce que** l'unité d'exploitation (7) et éventuellement les pièces supportant les électrodes (8) peut ou peuvent être enlevée(s) du fauteuil roulant.
